# EUROPÄISCHE PATENTSCHRIFT

(11) **EP 4 384 491 B1**
(45) Veröffentlichungstag und Bekanntmachung des Hinweises auf die Patenterteilung: **31.12.2025**
(21) Anmeldenummer: 22764731.0
(22) Anmeldetag: 11.08.2022
(51) Int. Cl.: C07C 37/055, C07C 39/07

(54) **VERFAHREN ZUR HERSTELLUNG VON KRESOL AUS DITOLYLETHER**
METHOD FOR THE PRODUCTION OF CRESOL FROM DITOLYL ETHER
PROCÉDÉ DE PRODUCTION DE CRÉSOL À PARTIR DE DITOLYLÉTHER

(30) Priorität: 13.08.2021 EP 21191354
(43) Veröffentlichungstag der Anmeldung: 19.06.2024
(73) Patentinhaber: LANXESS Deutschland GmbH, 50569 Köln (DE)
(72) Erfinder: SPRENGER, Paul, 51065 Köln (DE); KAESE, Thomas, 51375 Leverkusen (DE); HALLE, Olaf, 51061 Köln (DE); QUELLA, Hans-Jürgen, 51379 Leverkusen (DE); GILLESSEN, Eileen, 51371 Leverkusen (DE); KARTHAUS, Jürgen, 42697 Solingen (DE); NOTHEIS, Ulrich, 41539 Dormagen (DE)
(86) Internationale Anmeldenummer: PCT/EP2022/072585
(87) Internationale Veröffentlichungsnummer: WO 2023/017128

(56) Entgegenhaltungen:
- GAO FANG ET AL: "Chemo- and Regioselective Hydrogenolysis of Diaryl Ether C-O Bonds by a Robust Heterogeneous Ni/C Catalyst: Applications to the Cleavage of Complex Lignin-Related Fragments", vol. 55, no. 4, 22 January 2016 (2016-01-22), pages 1474 - 1478, XP055882368, ISSN: 1433-7851, Retrieved from the Internet <URL:https://onlinelibrary.wiley.com/doi/full-xml/10.1002/anie.201509133> [retrieved on 20220121], DOI: 10.1002/anie.201509133
- HUANG YAO-BING ET AL: "Selective hydrogenolysis of phenols and phenyl ethers to arenes through direct C-O cleavage over ruthenium-tungsten bifunctional catalysts", GREEN CHEMISTRY, vol. 17, no. 5, 1 January 2015 (2015-01-01), GB, pages 3010 - 3017, XP055882375, ISSN: 1463-9262, DOI: 10.1039/C5GC00326A

## Beschreibung

Die vorliegende Erfindung betrifft neue Verfahren zur Herstellung von Kresol aus Ditolylether.

### Stand der Technik

Es ist bekannt, dass die C-O-Bindungen aromatischer Ether mittels Hydrolyse bzw. Hydrogenolyse gespalten werden können beispielsweise in Gegenwart von radioaktiven Thoriumoxid (DE-A 2604474), was keine industrielle Anwendungsmöglichkeit eröffnet.

Des Weiteren ist der Einsatz von Nickel/ Nickelverbindungen auf diversen Trägern, wie Kohlenstoff oder Aluminiumoxid/Siliziumoxid, bei der Hydrogenolyse von C-O-Bindungen in Diarylethern in Gegenwart größerer Mengen sterisch anspruchsvoller starker Basen, wie z.B. NaOtBu oder Kaliumhexamethyldisilazid, bekannt, siehe Gao et al. Angew. Chem. Int. Ed. 2016, 55, 1474 -1478. Nachteil hierbei ist insbesondere die lange Reaktionszeit, der hohe Anteil an Übergangsmetall Nickel und aber auch der notwendige Einsatz sterisch anspruchsvoller starker Basen, die aufwändig abgetrennt werden müssen.

### Aufgabe der vorliegenden Erfindung

Aufgabe der vorliegenden Erfindung ist es daher, ein verbessertes Verfahren zur Herstellung von Kresol aus Ditolylether bereitzustellen, dass eine einfache Herstellung von Kresol in hoher Ausbeute und mit hoher Selektivität ermöglicht.

### Lösung der Aufgabe

Es wurde nun überraschend gefunden, dass die Aufgabe gelöst werden kann, wenn Ditolylether und Wasser in Gegenwart eines Katalysators umgesetzt werden, der aus mindestens 2 der folgenden Oxide Titanoxid, Zirkoniumoxid und Wolframoxid besteht und/oder dass ein Katalysator aus der Gruppe der Zeolithe eingesetzt wird, der zu mindestens 85 Gew.-% mindestens 2 der folgenden Oxide, ausgewählt aus Aluminiumoxid, Siliziumoxid, Titanoxid, Zirkoniumoxid und Wolframoxid, enthält.

### Gegenstand der Erfindung

Gegenstand der vorliegenden Erfindung ist ein Verfahren zur Herstellung von **Kresol** aus **Ditolylether** und **Wasser** in Gegenwart von **Katalysatoren,** die
aus mindestens 2 der folgenden Oxide Titanoxid, Zirkoniumoxid und Wolframoxid bestehen und/oder dass ein Katalysator aus der Gruppe der Zeolithe eingesetzt wird, der zu mindestens 85 Gew.-% mindestens 2 der folgenden Oxide, ausgewählt aus Aluminiumoxid, Siliziumoxid, Titanoxid, Zirkoniumoxid und Wolframoxid, enthält.

Dabei ist bevorzugt, dass ein Katalysator bestehend aus mindestens 2 der folgenden Oxide Titanoxid, Zirkoniumoxid und Wolframoxid und/oder ein Katalysator aus der Gruppe der Zeolithe eingesetzt wird, der zu mindestens 85 Gew.-% mindestens 2 der folgenden Oxide, ausgewählt aus Aluminiumoxid, Siliziumoxid, Titanoxid, Zirkoniumoxid und Wolframoxid, enthält.

**Kresol** im Sinne der Erfindung umfasst **o-Kresol, m-Kresol** und **p-Kresol** als Einzelverbindung sowie als Gemisch. Gemische können dabei in beliebigen Verhältnissen von o-, m-, und p-Kresol zueinander vorliegen.

**Ditolylether** im Sinne der Erfindung umfasst 2,2'-, 2,3'-, 2,4'-, 3,3'-, 3,4'- und/oder 4,4'-Ditolylether als einzelne Verbindung sowie als Mischung von 2 oder mehr dieser einzelnen vorgenannten Verbindungen.

Bevorzugt ist der Einsatz eines Gemisches aus **2,2'-, 2,3'-, 2,4'-, 3,3'-, 3,4'- und 4,4'-Ditolylether,** wie es beispielsweise bei der alkalischen Hydrolyse von Chlortoluolen anfällt (siehe H. Fiege, Cresols and Xylenols, Ullmann's Encyclopedia of Industrial Chemistry, Seite 427, Vol. 10, Wiley-VCH Verlag GmbH & Co. KGaA, Weinheim, 2012.)

**Wasser** im Sinne der Erfindung umfasst dabei vorzugsweise vollentsalztes Wasser.

**Katalysator** im Sinne der Erfindung bedeutet, dass einen Katalysator eingesetzt wird, der zu mindestens 85 Gew.-%, bevorzugt 90 - 100 Gew.-% mindestens 2 der folgenden Oxide, ausgewählt aus Aluminiumoxid, Siliziumoxid, Titanoxid, Zirkoniumoxid und Wolframoxid, enthält.

In einer bevorzugten Ausführungsform besteht der **Katalysator** aus mindestens 2 der folgenden Oxide Titanoxid, Zirkoniumoxid und Wolframoxid und/oder aus der Gruppe der Zeolithe, die zu mindestens 85 Gew.-% mindestens 2 der folgenden Oxide, ausgewählt aus Aluminiumoxid, Siliziumoxid, Titanoxid, Zirkoniumoxid und Wolframoxid, enthalten.

In einer bevorzugten Ausführungsform enthält der **Katalysator** aus der Gruppe der Zeolithe Siliziumoxid, Aluminiumoxid, Titanoxid und/oder Zirkoniumoxid.

Dabei ist bevorzugt, dass der Katalysator vorzugsweise Siliziumoxid und Aluminiumoxid enthält und vorzugsweise das molare Verhältnis von Silizium zu Aluminium 3:1 bis 300 :1 beträgt, besonders bevorzugt 5:1 bis 250:1, ganz besonders bevorzugt 30:1 bis 90:1 beträgt.

In einer besonders bevorzugten Ausführungsform hat der **Katalysator** die Struktur eines **Zeolithen.** Unter **Zeolith** im Sinne der Erfindung wird dabei vorzugsweise ein mikroporöses Material verstanden, dessen Struktur charakterisiert ist durch ein Gerüst aus eckenverknüpften Tetraedern. Jeder Tetraeder besteht in der Regel aus vier Sauerstoffatomen, die ein Kation umgeben. Bevorzugte Kationen für diese Tetraeder sind vorzugsweise aus Aluminium und Silicium.

Die Zeolithstruktur besteht bevorzugt aus Sauerstoff-Aluminium-Tetraedern und Sauerstoff-Silicium-Tetraedern.

Jede zeolithische Zellstruktur ist bekanntermaßen dabei über die Strukturkommision der Internationalen Zeolith Assosiation (IZA) über einen 3 Buchstabencode charakterisiert.

Besonders bevorzugt handelt es sich bei den Zeolithen um Mischungen aus Aluminiumoxid und Siliziumoxid der Gerüsttypen:
LTA = Linde Typ A (z. B.: Zeolith A),
MFI = Mobil Five (z.B.: ZSM-5, Zeolite Socony Mobil-5),
MOR = Mordenit,
BEA = Beta Zeolith und/oder
FAU = Faujasit (z.B.: Zeolith X,Y).

Ganz besonders bevorzugt als Gerüsttypen dabei sind MFI und MOR.

Der Zeolith kann als Pulver oder als Formkörper eingesetzt werden. Zur Formgebung als Formkörper wird der Zeolith bevorzugt mit 5-70 Gew.-% Aluminium- oder Silicium-basierten Bindematerials und verschiedener anorganischer und organischer Hilfsmittel, wie beispielsweise Salpetersäure, Zitronensäure, Essigsäure, Methylcellulose, Glycerol, Polyethylenglycol, Zucker, und/oder Stärke versetzt und verarbeitet. Verarbeitet umfasst in diesem Fall die Schritte Formgebung, z. B. durch Pressen, und Temperierung bei Temperaturen im Bereich von 350 bis 900 °C.

Für das erfindungsgemäße Verfahren werden aus der Gruppe der MFI-Zeolithe vorzugsweise handelsübliche ZSM-5-Zeolithe (Zeolite Socony Mobil-5) und MFI-Zeolithe beispielsweise der Fa. Clariant Produkte (Deutschland) GmbH eingesetzt.

Die ZSM-5 und MFI-Wirtstruktur zeichnet sich vorzugsweise durch zehngliedrige Ringe aus, die ein in drei Raumrichtungen verlaufendes und sich kreuzendes Porensystem bilden. Ferner gibt es, wie für "high-silica" Zeolithe und Pentasil-Zeolithe typisch, eine große Zahl an fünfgliedrigen Ringen (neben wenigen Vier-, Sechs-, Sieben- und Achtringen).

Die allgemeine chemische Zusammensetzung der Stoffgruppe der Zeolithe ist exemplarisch für Al- und Si-Oxid wie folgt:

Mⁿ⁺_{x/n} [(AlO₂)⁻ₓ (SiO₂)_{y}] · z H₂O

Mⁿ⁺ ist ein Kation, vorzugsweise H⁺, Na⁺ und/oder NH₄⁺, wobei n die Ladung des Kations bezeichnet und vorzugsweise 1 oder 2 ist. Die Kationen Mⁿ⁺ sind in der Regel nicht Teil der strukturgebenden Aluminium-Sauerstoff-Tetraeder und Silicium-Sauerstoff-Tetraeder, sondern befinden sich zum Ladungsausgleich in den Zeolith-Kanälen bzw. -Hohlräumen. In der bevorzugten Ausführungsform wird H⁺ als Kation eingesetzt, was in der Schreibweise H-ZSM-5 und H-MFI gekennzeichnet werden kann. Mittels chemischer Derivatisierung, z.B. über Ionenaustausch-Reaktionen, kann die Sorte des Kations reversibel verändert werden.

Das molare Verhältnis von Sauerstoff-Silicium-Tetraedern zu Sauerstoff-Aluminium-Tetraedern (kurz: Si/Al-Verhältnis) wird als Modul bezeichnet und durch den Quotient y/x ausgedrückt. In der bevorzugten Form liegt y/x bei 3-300, besonders bevorzugt 30-90. Der Quotient kann in der Schreibweise H-MFI-y/x, also z.B. H-MFI-90, gekennzeichnet werden.

Für das erfindungsmäßige Verfahren können handelsübliche Mischoxid-Katalysatoren als Formkörper eingesetzt werden, bevorzugt als Kugeln oder Extrudate. Solche Katalysatoren sind beispielsweise Gemische von ZrO₂/WO₃ und ZrO₂/SiO₂ der Firma Saint-Gobain Ceramic Materials GmbH, und Al₂O₃/SiO₂ von Shell Catalysts & Technologies Leuna GmbH. Als Gemische von Al₂O₃/SiO₂ können weiterhin handelsübliche Zeolithe eingesetzt werden, bevorzugt als Kugeln oder Extrudate. Beispiele für handelsübliche Zeolithe sind Extrudate von MFI-Zeolithen, MOR-Zeolithen, BEA-Zeolithen, wie beispielsweise von der Firma Clariant Produkte (Deutschland) GmbH, und FAU-Zeolithe, wie beispielsweise von der Firma Zeolyst International.

Das erfindungsgemäße Verfahren wird vorzugsweise bei Temperaturen von 250 bis 450°C, vorzugsweise 270 bis 400°C, besonders bevorzugt 300 bis 370 °C durchgeführt wird.

Das erfindungsgemäße Verfahren wird vorzugsweise bei einem Druck von 0,5 bar bis 300 bar, besonders bevorzugt 0,9 bar bis 50 bar und ganz besonders bevorzugt 1 bar bis 10 bar durchgeführt.

Das erfindungsgemäße Verfahren wird vorzugsweise in Reaktoren durchgeführt.

Als Reaktoren sind alle Behälter einsetzbar, die die Dosierung von Gasen ermöglichen und wo der Katalysator als starre Schüttung vorliegt, z.B. Festbettreaktoren, und solche, wo der Katalysator durch die Eduktzuspeisung oder ein Rühraggregat bewegt wird, z. B. Flugstromreaktoren, Wirbelschichtreaktoren oder Batch-Reaktoren. Die Edukte können gasförmig oder flüssig dosiert werden. In der bevorzugten Ausführung werden die Edukte als Gase in einen Festbettreaktor mit starrer Katalysatorschüttung dosiert. Das erfindungsgemäße Verfahren kann dabei kontinuierlich oder diskontinuierlich durchgeführt werden.

Die Gesamtmenge des Katalysators, bezogen auf Ditolylether (DTE), kann bei kontinuierlich betriebenen Reaktoren oder bei diskontinuierlich betriebenen Reaktoren beliebig gewählt werden. Für kontinuierlich betriebene Reaktoren beträgt dies vorzugsweise 0,01-1000 g DTE / (g Katalysator × h), besonders bevorzugt 0,1-100 g DTE / (g Katalysator × h). Bei diskontinuierlich betriebenen Reaktoren beträgt diese vorzugsweise 0,1-10000 g DTE / g Kat.

Das molare Verhältnis von **Ditolylether zu Wasser** beträgt vorzugsweise 10:1 bis 1:40, besonders bevorzugt 1:1 bis 1:20, ganz besonders bevorzugt 1:5 bis 1:15.

Zusätzlich zu Ditolylether und Wasser kann ein weiteres Gas, vorzugsweise ein Inertgas, wie Stickstoff oder Argon, dosiert werden. Vorzugsweise werden so viele Normliter Lₙ an Stickstoff oder Argon dosiert, dass der Anteil am Gesamtvolumen 0-95 Vol.-% beträgt, besonders bevorzugt 0 - 40 Vol.-%.

**Stickstoff** im Sinne der Erfindung hat vorzugsweise eine Reinheit größer 99 Vol.-%.

Bei dieser Ausführungsform des Verfahrens zur Herstellung von **Kresol** aus **Ditolylether** und **Wasser** in Gegenwart eines Katalysators spricht man auch von einer hydrolytischen Spaltung des Ditolylethers.

In dieser bevorzugten Ausführungsform der Erfindung ist folgende Durchführung des Verfahrens in kontinuierlicher Fahrweise bevorzugt:
Der **Katalysator** wird in einem Festbettreaktor vorgelegt und auf 300 °C bis 370 °C erwärmt. Ein auf mindestens 250 °C vorgewärmtes Gemisch aus Ditolylether und Wasser im molaren Verhältnis in einem Bereich 1:5 bis 1:15, versetzt mit höchstens 40 Vol.-% Stickstoff, wird mit 0,1 g bis 5 g Ditolylether pro g Katalysator und Stunde dosiert. Das gebildete Reaktionsprodukt wird aufgefangen, da dies in hoher Ausbeute gebildetes Kresol enthält.

In einer weiteren bevorzugten Ausführungsform der Erfindung erfolgt die Herstellung von **Kresol** aus **Ditolylether hydrogenolytisch** durch die Umsetzung in Gegenwart von Wasser, **Wasserstoff** und eines Katalysators, der zusätzlich mindestens ein Metall aus der Gruppe der **Platinmetalle** (Ru, Rh, Pd, Os, Ir, Pt) enthält.

Vorteil der **hydrogenolytischen** Herstellung von **Kresol** aus **Ditolylether** ist der gleichzeitige Anfall von Toluol.

Bei dieser bevorzugten Ausführungsform der Erfindung wird in Bezug auf **Kresol, Ditolylether, Wasser** auf die zuvor genannten Definitionen und Ausführungsformen verwiesen.

**Wasserstoff** im Sinne der Erfindung hat vorzugsweise eine Reinheit größer 99 Vol.-%.

**Katalysator** im Sinne der Erfindung bezüglich der Hydrogenolyse bedeutet, dass einen Katalysator eingesetzt wird, der zu mindestens 85 Gew.-%, bevorzugt 90 - 99,9 Gew.-% mindestens 2 der folgenden Oxide, ausgewählt aus Aluminiumoxid, Siliziumoxid, Titanoxid, Zirkoniumoxid und Wolframoxid, und zusätzlich mindestens ein Element aus der Gruppe der Platinmetalle enthält.

In einer bevorzugten Ausführungsform enthält der **Katalysator** Siliziumoxid und Aluminiumoxid oder Zirkoniumoxid und Wolframoxid und zusätzlich mindestens ein Element aus der Gruppe der Platinmetalle.

Dabei ist bevorzugt, dass das molare Verhältnis von Siliziumoxid zu Aluminiumoxid 3:1 bis 300:1 beträgt, besonders bevorzugt 5:1 bis 250:1, ganz besonders bevorzugt 30:1 bis 90:1 beträgt.

In einer besonders bevorzugten Ausführungsform hat der **Katalysator** die Struktur eines **Zeolithen.** In Bezug auf die Definition von **Zeolith** im Sinne der Erfindung wird dabei auf die obigen Ausführungen verweisen.

Besonders bevorzugt handelt es sich bei den Zeolithen um Mischungen aus Aluminiumoxid und Siliziumoxid der Gerüsttypen:
LTA = Linde Typ A (z. B.: Zeolith A),
MFI = Mobil Five (z.B.: ZSM-5, Zeolite Socony Mobil-5),
MOR = Mordenit,
BEA = Beta Zeolith und/oder
FAU = Faujasit (z.B.: Zeolith X,Y).

Ganz besonders bevorzugt als Gerüsttypen dabei sind MFI und MOR.

Für die Elemente aus der Gruppe der Platinmetalle ist ein Anteil von 0,1-10 Gew.-%, bezogen auf die Gesamtmenge des Katalysators, bevorzugt. Besonders bevorzugt ist für die Elemente aus der Gruppe der Platinmetalle ein Anteil von 0,5-5 Gew.-%.

Des Weiteren bevorzugt ist, dass es sich bei dem **Metall aus der Gruppe der Platinmetalle** um Platin und/oder Rhodium handelt.

**Platinmetallhaltige Katalysatoren** können durch gängige Herstellungsverfahren für edelmetallhaltige Katalysatoren hergestellt werden. Hierfür wird beispielsweise eine Metallsalzlösung des Edelmetalls mit dem Katalysatorgrundkörper durch Tränken oder Sprühen in Kontakt gebracht. Das Tränken kann als Tauchtränkung durchgeführt werden, bei der das Volumen an wässriger Lösung größer ist als das Flüssigkeitsaufnahmevolumen des zu belegenden Katalysatorgrundkörpers, oder als Trockentränkung, bei der das Volumen an wässriger Lösung kleiner oder gleich dem Flüssigkeitsaufnahmevolumen des zu belegenden Katalysatorgrundkörpers ist. Gleiches gilt für das Aufsprühen von Edelmetalllösungen auf den Katalysatorgrundkörper. Ferner kann ein Ionenaustauschprozess durchgeführt werden, um Edelmetalle den Katalysatoren hinzuzufügen, beispielsweise indem eine verdünnte Metallsalzlösung des Edelmetalls mehrfach über einen Katalysatorgrundkörper dosiert wird. Im Anschluss an eine Tränkung, Sprühung oder einen Ionenaustausch erfolgt die Trocknung des edelmetallhaltigen Katalysators, bevorzugt in einem Luftstrom von erhöhter Temperatur, bevorzugt bei 60 °C bis 200 °C für 0,5 h bis10 h. Optional kann auch eine Kalzination bei erhöhter Temperatur erfolgen, bevorzugt bei 200 °C bis 1000 °C für 10 min bis 24 h.

Das erfindungsgemäße Verfahren wird vorzugsweise bei **Temperaturen** von 250 bis 450°C, besonders bevorzugt 270 bis 400°C, ganz besonders bevorzugt 300 bis 370 °C durchgeführt wird.

Das erfindungsgemäße Verfahren wird vorzugsweise bei einem **Druck** von 0,5 bar bis 300 bar, besonders bevorzugt 0,9 bar bis 50 bar und ganz besonders bevorzugt 1 bar bis 10 bar durchgeführt.

Das erfindungsgemäße Verfahren wird vorzugsweise in **Reaktoren** durchgeführt.

Als **Reaktoren** sind alle **Behälter** einsetzbar, die die Dosierung von Gasen ermöglichen und wo der Katalysator als starre Schüttung vorliegt, z.B. Festbettreaktoren, und solche, wo der Katalysator durch die Eduktzuspeisung oder ein Rühraggregat bewegt wird, z. B. Flugstromreaktoren, Wirbelschichtreaktoren oder Batch-Reaktoren. Die Edukte können gasförmig oder flüssig dosiert werden. In der bevorzugten Ausführung werden die Edukte als Gase in einen Festbettreaktor mit starrer Katalysatorschüttung dosiert. Das erfindungsgemäße Verfahren kann dabei kontinuierlich oder diskontinuierlich durchgeführt werden.

Die Gesamtmenge des Katalysators, bezogen auf Ditolylether (DTE), kann bei kontinuierlich betriebenen Reaktoren oder bei diskontinuierlich betriebenen Reaktoren beliebig gewählt werden. Für kontinuierlich betriebene Reaktoren beträgt dies vorzugsweise 0,01-1000 g DTE / (g Katalysator × h), besonders bevorzugt 0,1-100 g DTE / (g Katalysator × h). Bei diskontinuierlich betriebenen Reaktoren beträgt diese vorzugsweise 0,1-10000 g DTE / g Kat.

Das molare Verhältnis von **Ditolylether zu Wasser** beträgt vorzugsweise 10:1 bis 1:40, besonders bevorzugt 1:1 bis 1:20, ganz besonders bevorzugt 1:5 bis 1:15.

Das molare Verhältnis von **Ditolylether zu Wasserstoff** beträgt vorzugsweise 10:1 bis 1:100, besonders bevorzugt 1:1 bis 1:50, ganz besonders bevorzugt 1:5 bis 1:40.

Zusätzlich zu **Ditolylether** und **Wasser** und **Wasserstoff** kann ein weiteres Gas, z. B.: ein Inertgas, wie Stickstoff oder Argon, dosiert werden. Vorzugsweise werden so viele Normliter Lₙ an Stickstoff oder Argon dosiert, dass der Anteil am Gesamtvolumen 0-95 Vol.-% beträgt, besonders bevorzugt 0-40 Vol.-%.

Als **Reaktoren** sind alle **Behälter** einsetzbar, die die Dosierung von Gasen ermöglichen und wo der Katalysator als starre Schüttung vorliegt, z.B. Festbettreaktoren, und solche, wo der Katalysator durch die Eduktzuspeisung oder ein Rühraggregat bewegt wird, z. B. Flugstromreaktoren, Wirbelschichtreaktoren oder Batch-Reaktoren. Die Edukte können gasförmig oder flüssig dosiert werden. In der bevorzugten Ausführung werden die Edukte als Gase in einen Festbettreaktor mit starrer Katalysatorschüttung dosiert.

In dieser bevorzugten Ausführungsform der Erfindung als Hydrogenolyse ist folgende Durchführung des Verfahrens in kontinuierlicher Fahrweise bevorzugt:
Der **Katalysator** wird in einem Festbettreaktor vorgelegt und auf 300 °C bis 370 °C erwärmt. Ein auf mindestens 250 °C vorgewärmtes Gemisch aus Ditolylether, Wasser und Wasserstoff im molaren Verhältnis von Ditolylether zu Wasser in einem Bereich 1:5 bis 1:15 und im molaren Verhältnis von Ditolylether zu Wasserstoff im Bereich von 1:5 bis 1:40, versetzt mit höchstens 40 Vol.-% Stickstoff, wird mit 0,1 g bis 5 g Ditolylether pro g Katalysator und Stunde dosiert. Das gebildete Reaktionsprodukt wird aufgefangen, da dies in hoher Ausbeute gebildetes Kresol enthält.

Das erfindungsgemäße Verfahren wird anhand der nachfolgenden Beispiele erläutert, ohne dabei auf diese beschränkt zu sein.

### Beispiele

Die nachstehend aufgeführten Versuche wurden in einem Stahlrohr mit Bodenlochplatte als Reaktor durchgeführt. Angaben zu Art und Hersteller des Katalysators je Versuch finden sich in Tabelle 1 und Tabelle 2. In den Reaktor wurde ein gasförmiges Gemisch aus Ditolylether, Wasser und Stickstoff und/oder Wasserstoff in den in Tabelle 3 und 4 angegeben Verhältnissen eingeleitet. Nach der Reaktion wurde das Produktgemisch auf Raumtemperatur abgekühlt und Aceton hinzudosiert, um ein einphasiges Gemisch zu erhalten, das mittels Gaschromatographie-Flammenionisation analysiert wurde. Die Ergebnisse sind in den Tabellen 3 und 4 aufgelistet.

**Tabelle 1: Eingesetzte Katalysatoren (KAT)**

| Nr. | KAT | Zusammensetzung | Si/ Al-Verhältnis | Handelsname des jeweiligen KATs, vor Aufbringen des Edelmetalls |
|---|---|---|---|---|
| 1 (V) | Al₂O₃ | Al₂O₃ | - | Alumina Spheres 1.8/210, Sasol Germany GmbH |
| 2 (E) | ZrO₂, 18 Gew.-% WO₃ | ZrO₂/ WO₃ | - | SZ 61143, Saint-Gobain Ceramic Materials GmbH |
| 3 (E) | H-MFI-30 | SiO₂/Al₂O₃ | 30:1 | Clariant Produkte (Deutschland) GmbH |
| 4 (E) | H-MFI-55 | SiO₂/Al₂O₃ | 55:1 | Süd-Chemie AG |
| 5 (E) | H-MFI-60 | SiO₂/Al₂O₃ | 60:1 | Süd-Chemie AG |
| 6 (E) | H-MFI-90 | SiO₂/Al₂O₃ | 90:1 | HCZP 90E, Clariant Produkte (Deutschland) GmbH |
| 7 (E) | H-MOR-40 | SiO₂/Al₂O₃ | 40:1 | HCZM 40E, Clariant Produkte Deutschland) GmbH |
| 8 (E) | H-MFI-30, 0,5 Gew.-% Platin | SiO₂/Al₂O₃ | 30:1 | Clariant Produkte (Deutschland) GmbH |
| 9 (E) | H-MFI-55, 0,5 Gew.-% Platin | SiO₂/Al₂O₃ | 55:1 | Süd-Chemie AG |
| 10 (E) | H-MFI-90, 0,5 Gew.-% Platin | SiO₂/Al₂O₃ | 90:1 | HCZP 90E, Clariant Produkte (Deutschland) GmbH |
| 11 (E) | H-MFI-240, 0,5 Gew.-% Platin | SiO₂/Al₂O₃ | 240:1 | Clariant Produkte (Deutschland) GmbH |
| 12 (E) | H-MFI-55, 1 Gew.-% Platin | SiO₂/Al₂O₃ | 55:1 | Süd-Chemie AG |
| 13 (E) | H-MOR-40, 1 Gew.-% Platin | SiO₂/Al₂O₃ | 40:1 | HCZM 40E, Clariant Produkte (Deutschland) GmbH |
| 14 (E) | H-MFI-90, 1 Gew.-% Rhodium | SiO₂/Al₂O₃ | 90:1 | HCZP 90E, Clariant Produkte (Deutschland) GmbH |
| 15 (E) | H-MFI-60, 1 Gew.-% Platin, 0,2 Gew.-% Nickel | SiO₂/Al₂O₃ | 60:1 | Süd-Chemie AG |
| 16 (V) | Al₂O₃/SiO₂ mit 20 Gew.-% Nickel | SiO₂/Al₂O₃ | 1:5 | Basierend auf Bsp. 6 für Diphenylether in Gao et al. Angew. Chem. Int. Ed. 2016, 55, 1474 -1478, ohne Zusatz von Basen |
| 17 (V) | Aktivkohle Norit RX 1.5 Extra, 0,1 Gew.-% Platin | C | - | Norit RX 1.5 Extra, Cabot Norit Nederland B.V. |

| | | | | |
|---|---|---|---|---|
| MFI = SiO₂/Al₂O₃-Zeolith; MOR = SiO₂/Al₂O₃-Zeolith mit Mordenit-Struktur E = erfindungsgemäß, V = Vergleichsbeispiel | | | | |

Das Edelmetall bei den edelmetallhaltigen Katalysatoren (siehe Beispiele 8 (E) bis 15 (E) wurde dabei - wie nachstehend erläutert - mittels Trockentränkung bzw. Ionentausch aufgebracht. Mengen und Eigenschaften sind Tabelle 2 zu entnehmen.

Für die Trockentränkung wurde zu einem Träger die in der Tabelle 2 angegebene Edelmetallquelle gelöst in vollentsalztem Wasser gegeben. Die Menge des vollentsalzten Wassers entspricht 98% der für den jeweiligen Träger geltenden Saugfähigkeit (siehe Tabelle 2) Nachdem die Lösung vollständig aufgenommen war, wurde der getränkte Träger für 1 h bei 120 °C im Warmluftstrom getrocknet und - mit Ausnahme von Beispiel 16 (V)- im statischen Ofen für 12 - 16 Stunden bei Temperaturen von 300 - 500°C kalziniert. Im Fall von Beispiel 16 (V) wurden Trockentränkung und Trocknung dreimal wiederholt, um die gesamte Menge an Edelmetalllösung aufbringen zu können.

Für den Ionenaustausch wurde zu einem Träger in ein Glasrohr mit Glasfrittenboden die Edelmetall-Dotierlösung gegeben und die Lösung 24 h über den Träger im Kreis gepumpt. Der Träger war währenddessen immer mit Flüssigkeit bedeckt.

**Tabelle 2: Mengen und Eigenschaften der edelmetallhaltigen Katalysatoren**

| Nr. | Träger vor Edelmetalldotierung | | | Dotierung mit Edelmetall | | |
|---|---|---|---|---|---|---|
| | Masse/g | Wassergehalt / Gew.-% | Saugfähigkeit / Gew.-% | Typ | Volumen der Lösung / mL | Edelmetallquelle |
| 8 (E) | 51,5 | 3,4 | 43,2 | Trockentränkung | 22,2 | 1 g H₂PtCl₆-Lösung, 25 Gew.-% Pt |
| 9 (E) | 51,7 | 3,8 | 40,5 | Trockentränkung | 20,9 | 1 g H₂PtCl₆-Lösung, 25 Gew.-% Pt |
| 10 (E) | 101,7 | 2,2 | 42,5 | lonenaustausch | 150,0 | 0,9 g [Pt(NH₃)₄]Cl₂×H₂O |
| 11 (E) | 51,4 | 3,3 | 51,5 | Trockentränkung | 25,6 | 1 g H₂PtCl₆-Lösung, 25 Gew.-% Pt |
| 12 (E) | 102,9 | 3,8 | 40,5 | lonenaustausch | 150,0 | 1,8 g [Pt(NH₃)₄Cl₂×H₂O |
| 13 (E) | 106,4 | 7,0 | 38,2 | Trockentränkung | 40,7 | 1 g H₂PtCl₆-Lösung, 25 Gew.-% Pt |
| 14 (E) | 50,6 | 2,2 | 42,5 | Trockentränkung | 21,1 | 5 g Rh(NO₃)₃-Lösung, 10 Gew.-% Rh |
| 15 (E) | 51,9 | 4,7 | 46,0 | Trockentränkung | 22,7 | 2 g H₂PtCl₆-Lösung, 25 Gew.-% Pt + 0,5 g Ni(NO₃)₂×6H₂O |
| 16 (V) | 240,0 | 0,8 | 44,0 | Trockentränkung | 320,0 | 291 g Ni(NO₃)₂×6H₂O |
| 17 (V) | 72,2 | 3,2 | 92,8 | Trockentränkung | 67,0 | 0,3 g H₂PtCl₆-Lösung, 25 Gew.-% Pt |

**Tabelle 3: Messergebnisse für die hydrolytische Spaltung von Ditolylether bei einer Temperatur von 315 °C, einem Druck von 1 bar und einem Katalysatorvolumen von 68 mL. Die Flüsse betrugen: 15,2 g/h Ditolylether und 13,8 g/h Wasser mit einem Anteil von 20 Vol.-% Stickstoff.**

| Nr. | Katalysator | Umsatz DTE /% | Selektivität Kresol /% | Ausbeute Kresol /% |
|---|---|---|---|---|
| 1 (V) | Al₂O₃ | 1,3 | 51,9 | 0,7 |
| 2 (E) | ZrO₂, 18 Gew.-% WO₃ | 23,1 | 54,0 | 12,5 |
| 3 (E) | H-MFI-30 | 13,4 | 74,2 | 9,9 |
| 4 (E) | H-MFI-55 | 12,8 | 68,2 | 8,7 |
| 5 (E) | H-MFI-60 | 14,3 | 70,5 | 10,1 |
| 6 (E) | H-MFI-90 | 7,4 | 84,6 | 6,2 |
| 7 (E) | H-MOR-40 | 15,5 | 46,4 | 7,2 |

| | | | | |
|---|---|---|---|---|
| V= Vergleich, E = erfindungsgemäß | | | | |

Bei den nach dem erfindungsgemäßen Verfahren durchgeführten Versuchen 2-7 mit Wasser zeigte sich ein hoher Umsatz, eine hohe Selektivität und eine hohe Ausbeute im Vergleich zu dem Vergleichsbeispiel, Beispiel 1 (V).

**Tabelle 4: Messergebnisse für die hydrogenolytische Spaltung von Ditolylether bei einer Temperatur von 315 °C, einem Druck von 1 bar und einem Katalysatorvolumen von 68 mL. Die Flüsse betrugen: 15,2 g/h Ditolylether und 13,8 g/h Wasser mit einem Anteil von 20 Vol.-% Wasserstoff.**

| Nr. | Katalysator | Umsatz DTE / % | Selektiviät Kresol / % | Selektiviät Toluol / % | Ausbeute Kresol + Toluol /% |
|---|---|---|---|---|---|
| 8 (E) | H-MFI-30, 0,5 Gew.-% Platin | 93,3 | 21,9 | 41,7 | 59,4 |
| 9 (E) | H-MFI-55, 0,5 Gew.-% Platin | 95,3 | 22,3 | 32,2 | 51,9 |
| 10 (E) | H-MFI-90, 0,5 Gew.-% Platin | 86,9 | 22,1 | 40,1 | 54,1 |
| 11 (E) | H-MFI-240, 0,5 Gew.-% Platin | 98,1 | 20,7 | 37,3 | 56,9 |
| 12 (E) | H-MFI-55, 1 Gew.-% Platin | 85,4 | 32,4 | 43,4 | 64,8 |
| 13 (E) | H-MOR-40, 1 Gew.-% Platin | 94,2 | 17,7 | 35,1 | 49,7 |
| 14 (E) | H-MFI-90, 1 Gew.-% Rhodium | 36,3 | 35,1 | 39,4 | 27,1 |
| 15 (E) | H-MFI-60, 1 Gew.-% Platin, 0,2 Gew.-% Nickel | 96,5 | 16,7 | 32,2 | 47,3 |
| 16 (V) ¹⁾ | Al₂O₃/SiO₂ mit 20 Gew.-% % Nickel | 1,8 | 16,3 | 5,1 | 0,4 |
| 17 (V) | Aktivkohle Norit RX 1.5 Extra, 0,1 Gew.-% Platin | 4,4 | 32,1 | 42,0 | 3,2 |

| | | | | | |
|---|---|---|---|---|---|
| V= Vergleichsversuche, E= erfindungsgemäß ¹⁾ analog zu Gao et al. Angew. Chem. Int. Ed. 2016, 55, 1474 -1478. | | | | | |

Es zeigte sich, dass bei den nach dem erfindungsgemäßen Verfahren durchgeführten Versuchen 8 (E) - 13 (E) und 15 (E) für platinhaltige bzw. im Fall von Beispiel 14 (E) für rhodiumhaltige Katalysatoren ein hoher Umsatz bezogen auf Ditolylether und eine hohe Selektivität und Ausbeute bezogen auf Kresol und Toluol erhalten wird, im Vergleich zu den Vergleichsbeispielen 16 (V) und 17 (V).

Vergleichsbeispiel 16 (V) zeigt zudem, dass der dem Stand der Technik entsprechende Einsatz von Nickel ohne den Zusatz einer starken Base einen extrem geringen Umsatz, eine deutlich geringe Selektivität und Ausbeute ergibt, als die mindestens ein Element der Platingruppe enthaltenden erfindungsgemäßen Katalysatoren 8 (E) - 15 (E).

## Patentansprüche

1. Verfahren zur Herstellung von Kresol aus Ditolylether und Wasser in Gegenwart eines Katalysators, **dadurch gekennzeichnet, dass** ein Katalysator bestehend aus mindestens 2 der folgenden Oxide Titanoxid, Zirkoniumoxid und Wolframoxid und/oder ein Katalysator aus der Gruppe der Zeolithe eingesetzt wird, der zu mindestens 85 Gew.-% mindestens 2 der folgenden Oxide, ausgewählt aus Aluminiumoxid, Siliziumoxid, Titanoxid, Zirkoniumoxid und Wolframoxid, enthält.

2. Verfahren gemäß Anspruch 1, **dadurch gekennzeichnet, dass** der Katalysator aus der Gruppe der Zeolithe Siliziumoxid, Aluminiumoxid, Titanoxid und/oder Zirkoniumoxid enthält.

3. Verfahren gemäß Anspruch 1 oder 2, **dadurch gekennzeichnet, dass** der Katalysator aus der Gruppe der Zeolithe Siliziumoxid und Aluminiumoxid enthält und das molare Verhältnis von Silizium zu Aluminium 3:1 bis 300:1 beträgt, bevorzugt 5:1 bis 250:1, besonders bevorzugt 30:1 bis 90:1.

4. Verfahren nach einem der Ansprüche 1 bis 3, **dadurch gekennzeichnet, dass** als Katalysatoren Zeolithe der Typen LTA, MFI, MOR, BEA, FAU eingesetzt werden, besonders bevorzugt MFI, MOR.

5. Verfahren nach einem der Ansprüche 1 bis 4, **dadurch gekennzeichnet, dass** der Katalysator zusätzlich mindestens ein Element aus der Gruppe der Platinmetalle enthält und die Reaktion in Gegenwart von Wasserstoff durchgeführt wird.

6. Verfahren nach einem der Ansprüche 1 bis 5, **dadurch gekennzeichnet, dass** der Anteil der Elemente aus der Gruppe der Platinmetalle 0,1-10 Gew.-%, bezogen auf die Gesamtmenge des Katalysators beträgt, besonders bevorzugt 0,5-5 Gew.-%.

7. Verfahren nach einem der Ansprüche 5 bis 6, **dadurch gekennzeichnet, dass** es sich bei dem Metall aus der Gruppe der Platinmetalle um Platin und/oder Rhodium handelt.

8. Verfahren nach einem der Ansprüche 1 bis 7, **dadurch gekennzeichnet, dass** dieses bei Temperaturen von 250 bis 450 °C, vorzugsweise 270 bis 400 °C, besonders bevorzugt 300 bis 370 °C durchgeführt wird.

9. Verfahren nach einem der Ansprüche 1 bis 7, **dadurch gekennzeichnet, dass** dieses bei einem Druck von 0,5 bar bis 300 bar, besonders bevorzugt 0,9 bar bis 50 bar und ganz besonders bevorzugt 1 bar bis 10 bar durchgeführt.

## Claims

1. Process for producing cresol from ditolyl ether and water in the presence of a catalyst, **characterized in that** a catalyst consisting of at least 2 of the following oxides titanium oxide, zirconium oxide and tungsten oxide and/or a catalyst from the group of zeolites which contains at least 85% by weight of at least 2 of the following oxides selected from aluminium oxide, silicon oxide, titanium oxide, zirconium oxide and tungsten oxide is employed.

2. Process according to Claim 1, **characterized in that** the catalyst from the group of zeolites contains silicon oxide, aluminium oxide, titanium oxide and/or zirconium oxide.

3. Process according to Claim 1 or 2, **characterized in that** the catalyst from the group of zeolites contains silicon oxide and aluminium oxide and the molar ratio of silicon to aluminium is 3:1 to 300:1, preferably 5:1 to 250:1, particularly preferably 30:1 to 90:1.

4. Process according to any of Claims 1 to 3, **characterized in that** the catalysts employed are zeolites of the types LTA, MFI, MOR, BEA, FAU, particularly preferably MFI, MOR.

5. Process according to any of Claims 1 to 4, **characterized in that** the catalyst additionally contains at least one element from the group of platinum metals and the reaction is performed in the presence of hydrogen.

6. Process according to any of Claims 1 to 5, **characterized in that** the proportion of the elements from the group of platinum metals is 0.1-10% by weight, based on the total amount of the catalyst, particularly preferably 0.5-5% by weight.

7. Process according to either of Claims 5 to 6, **characterized in that** the metal from the group of platinum metals is platinum and/or rhodium.

8. Process according to any of Claims 1 to 7, **characterized in that** it is performed at temperatures of 250°C to 450°C, preferably 270°C to 400°C, particularly preferably 300°C to 370°C.

9. Process according to any of Claims 1 to 7, **characterized in that** it is performed at a pressure of 0.5 bar to 300 bar, particularly preferably 0.9 bar to 50 bar and very particularly preferably 1 bar to 10 bar.

## Revendications

1. Procédé de fabrication de crésol à partir d'éther ditolylique et d'eau en présence d'un catalyseur, **caractérisé en ce qu'**on utilise un catalyseur constitué d'au moins 2 des oxydes suivants oxyde de titane, oxyde de zirconium et oxyde de tungstène et/ou un catalyseur du groupe des zéolithes, qui contient au moins 85 % en poids d'au moins 2 des oxydes suivants, choisis parmi l'oxyde d'aluminium, l'oxyde de silicium, l'oxyde de titane, l'oxyde de zirconium et l'oxyde de tungstène.

2. Procédé selon la revendication 1, **caractérisé en ce que** le catalyseur contient, parmi le groupe des zéolithes, de l'oxyde de silicium, de l'oxyde d'aluminium, de l'oxyde de titane et/ou de l'oxyde de zirconium.

3. Procédé selon la revendication 1 ou 2, **caractérisé en ce que** le catalyseur contient, parmi le groupe des zéolithes, de l'oxyde de silicium et de l'oxyde d'aluminium, et le rapport molaire entre le silicium et l'aluminium est de 3:1 à 300:1, de préférence de 5:1 à 250:1, et de manière particulièrement préférée de 30:1 à 90:1.

4. Procédé selon l'une quelconque des revendications 1 à 3, **caractérisé en ce que** l'on utilise comme catalyseurs des zéolithes des types LTA, MFI, MOR, BEA, FAU, de manière particulièrement préférée MFI, MOR.

5. Procédé selon l'une quelconque des revendications 1 à 4, **caractérisé en ce que** le catalyseur contient en outre au moins un élément du groupe des métaux du platine et la réaction est réalisée en présence d'hydrogène.

6. Procédé selon l'une quelconque des revendications 1 à 5, **caractérisé en ce que** la proportion des éléments du groupe des métaux du platine est de 0,1 à 10 % en poids par rapport à la quantité totale du catalyseur, de manière particulièrement préférée de 0,5 à 5 % en poids.

7. Procédé selon l'une quelconque des revendications 5 à 6, **caractérisé en ce que** le métal du groupe des métaux du platine est le platine et/ou le rhodium.

8. Procédé selon l'une quelconque des revendications 1 à 7, **caractérisé en ce qu'**il est mis en œuvre à des températures de 250 à 450 °C, de préférence de 270 à 400 °C, de manière particulièrement préférée de 300 à 370 °C.

9. Procédé selon l'une quelconque des revendications 1 à 7, **caractérisé en ce qu'**il est mis en œuvre à une pression de 0,5 bar à 300 bars, de manière particulièrement préférée de 0,9 bar à 50 bars et de manière tout particulièrement préférée de 1 bar à 10 bars.
